(19) Europäisches Patentamt
European Patent Office
Office européen des brevets

(11) Publication number: **0 461 809 B1**

# (12) EUROPEAN PATENT SPECIFICATION

(45) Date of publication of patent specification: **07.12.94**

(51) Int. Cl.⁵: **C07C 261/04**, A01N 47/40

(21) Application number: **91305115.7**

(22) Date of filing: **06.06.91**

The file contains technical information submitted after the application was filed and not included in this specification

## (54) Dibenzoylalkylcyanohydrazines.

(30) Priority: **14.06.90 US 538297**

(43) Date of publication of application:
**18.12.91 Bulletin 91/51**

(45) Publication of the grant of the patent:
**07.12.94 Bulletin 94/49**

(84) Designated Contracting States:
**AT BE CH DE DK ES FR GB GR IT LI LU NL SE**

(56) References cited:
**EP-A- 0 286 746**
**EP-A- 0 358 856**
**EP-A- 0 361 645**

(73) Proprietor: **ROHM AND HAAS COMPANY**
**Independence Mall West**
**Philadelphia Pennsylvania 19105 (US)**

(72) Inventor: **Phat Le, Dat**
**125 Washington Avenue**
**North Wales, Pennsylvania 19454 (US)**
Inventor: **Carlson, Glenn Richard**
**305 Britt Road**
**North Wales Pennsylvania 19454 (US)**

(74) Representative: **Smith, Julian Philip Howard et al**
**Rohm and Haas (UK) Limited,**
**European Operations Patent Dept.,**
**Lennig House,**
**2 Masons Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to novel dibenzoylalkylcyanohydrazines which are useful as pesticides, compositions containing those compounds, methods of controlling pests and processes for preparing the compounds of the present invention.

The search for pesticides which have a combination of excellent insecticidal activity and essentially no other toxicity is a continuing one because of factors such as the desire for compounds exhibiting greater activity, better selectivity, low undesirable environmental impact, and effectiveness against insects resistant to many known insecticides.

Certain dibenzoylalkylcyanohydrazines have been disclosed as insecticidal compounds. US-A-4,857,550 discloses insecticidal dibenzoyl-tert-butylcarbazonitriles compounds and methods for the preparation thereof. No compounds of the present invention are disclosed in this document. We have now found a class of compounds that show unexpected superior properties over the prior disclosed compounds.

In accordance with the present invention, there are provided compounds having the formula:

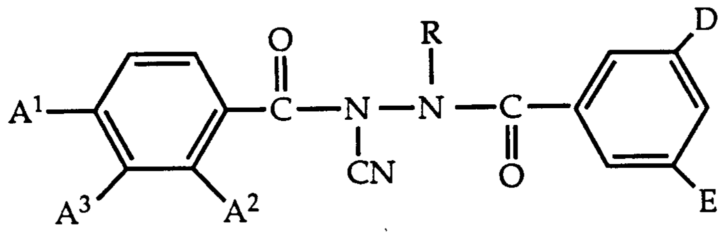

wherein R is t-butyl, methylneopentyl or neopentyl;

$A^1$ is hydrogen, methyl, ethyl, n-propyl or isopropyl;

when $A^1$ is hydrogen then $A^2$ and $A^3$ are each independently methyl, ethyl, methoxy, bromo, chloro or fluoro, and when $A^1$ is other than hydrogen, $A^2$ and $A^3$ are both hydrogen;

and D and E are each independently hydrogen, methyl, chloro, bromo or fluoro.

One class of compounds according to the invention are compounds of the formula

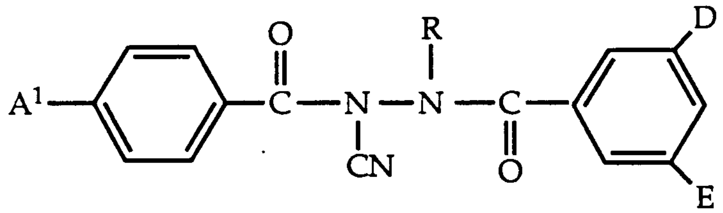

wherein R is t-butyl, methylneopentyl or neopentyl, $A^1$ is methyl, ethyl, n-propyl or isopropyl, and D and E are each independently hydrogen, methyl, chloro, bromo or fluoro.

In preferred compounds of this class, R is t-butyl.

In one embodiment of this class of compounds, $A^1$ is methyl, ethyl, n-propyl or isopropyl, D is methyl and E is hydrogen or methyl.

More preferred are compounds wherein $A^1$ is methyl, ethyl, n-propyl or isopropyl; D is methyl; and E is methyl.

Most preferred are compounds wherein $A^1$ is methyl or ethyl and D and E are methyl.

In another embodiment of this class, $A^1$ is methyl, ethyl, n-propyl or isopropyl and D and E are each hydrogen.

In yet another embodiment of this class, $A^1$ is methyl, ethyl n-propyl or isopropyl, and D and E are each independently bromo or chloro. Preferably, $A^1$ is methyl or ethyl and D and E are both chloro.

In another class of compounds of the invention are compounds of the formula

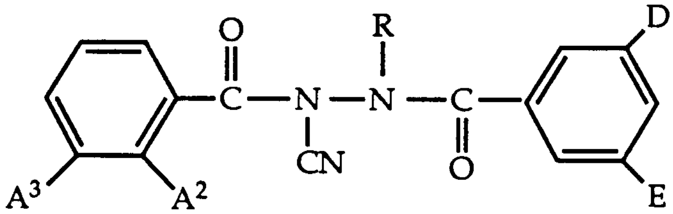

wherein

R is t-butyl, methylneopentyl or neopentyl

$A^2$ and $A^3$ are each independently methyl, ethyl, methoxy, chloro, bromo or fluoro; and

D and E are each independently hydrogen, methyl, chloro, bromo and fluoro.

Preferably, R is t-butyl; it is also preferred that $A^2$ and $A^3$ are each methyl and D and E are independently hydrogen or methyl.

Most preferably $A^2$, $A^3$ and D are methyl and E is hydrogen or methyl.

Compounds of the present invention are suitable for controlling plant destructive insects in crops of cultivated plants, ornamentals and forestry.

The compounds of the invention may be prepared from N,N'-dibenzoyl-N-alkylhydrazines of the formula

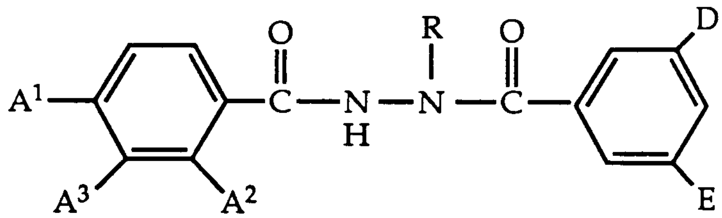

wherein $A^1$, $A^2$, $A^3$, D, E and R are as defined above, by reacting the N,N'-dibenzoyl-N-alkylhydrazines with an alkali metal hydride such as sodium hydride and a cyanogen halide such as cyanogen bromide. The reaction is generally carried out in an anhydrous organic solvent such as tetrahydrofuran, ether, methylene chloride or dioxane, preferably tetrahydrofuran at a temperature from about 0 ° C to about 100 ° C, preferably from about 15 ° C to about 50 ° C.

Alternatively, the reaction is carried out in a two-phase system comprising of an organic solvent non-miscible with water and a basic aqueous phase at a temperature of from about 5 ° C to about 95 ° C, preferably from about 15 ° C to about 50 ° C. Preferred organic solvents include methylene chloride and chloroform. The aqueous phase is preferably an alkali metal hydroxide such as 50% sodium hydroxide. In a two-phase system, a phase transfer catalyst such as tetrabutylammonium hydrogen sulfate or benzyltrimethylammonium chloride must be also be present.

The N,N'-dibenzoyl-N-alkylhydrazines which are starting materials in the above reactions are prepared by several processes.

In one process, a substituted or unsubstituted benzoyl halide (Reactant A) is reacted with t-butylhydrazine, methylneopentylhydrazine or neopentylhydrazine or a corresponding acid salt such as the hydrochloride or hydrobromide in the presence of a base in an inert or substantially inert solvent or mixture of solvents to yield an intermediate monobenzoylated alkylhydrazine which can be isolated or further reacted with another benzoyl halide in the presence of a base in an inert or substantially inert solvent or mixture of solvents to yield the N,N'-dibenzoyl-N'-alkylhydrazine.

Suitable solvents for use in the above processes include water; alcohols such as methanol, ethanol, and isopropanol; hydrocarbons such as toluene, xylene, hexane and heptane; glyme; tetrahydrofuran; acetonitrile; pyridine; or haloalkanes such as methylene chloride or mixtures of these solvents.

Preferred solvents are water, toluene, methylene chloride or a mixture of these solvents.

Examples of bases for use in the above processes include tertiary amines such as triethylamine; pyridine; potassium carbonate; sodium carbonate; sodium bicarbonate; sodium hydroxide; or potassium

hydroxide. Preferred bases are sodium hydroxide, potassium hydroxide or triethylamine.

The above process can be carried out at a temperature from about -20 °C to about 100 °C. Preferably, these reactions are carried out between about -5 °C and about 50 °C.

In another process, t-butylhydrazine, methylneopentylhydrazine or neopentylhydrazine or a corresponding acid addition salt, such as the hydrochloride or hydrobromide, is reacted with a dicarbonate such as di-t-butyldicarbonate, diethyldicarbonate, diphenyldicarbonate or dibenzyldicarbonate in the presence of a base in an inert or substantially enert solvent or mixture of solvents to yield an intermediate N-alkoxycarbonyl-N'-alkylhydrazine. This compound is then further reacted with a benzoyl halide in the presence of a base in an inert or substantially inert solvent or mixture of solvents to yield a N-alkoxycarbonyl-N'-alkyl-N'-benzoylhydrazine which is then further reacted with an acid in an inert or substantially inert solvent or mixture of solvents to yield a N'-alkyl-N'-benzoylhydrazine. This compound is then reacted with a benzoyl halide in the presence of a base in an inert or substantially inert solvent or mixture of solvents to yield the desired N,N'-dibenzoyl-N'-alkylhydrazine.

Suitable solvents for the first, second and fourth steps of the above process include water; tetrahydrofuran; dioxane; toluene; alcohols such as methanol, ethanol and isopropanol; hexane, acetonitrile; pyridine; and haloalkanes such as methylene chloride; or mixtures of these solvents. Preferred solvents are dioxane; toluene; tetrahydrofuran; pyridine; methylene chloride or water. Most preferred solvents are dioxane; water; or toluene.

Examples of the bases for use in the first, second and fourth steps of this process include tertiary amines such as triethylamine; pyridine; potassium carbonate; sodium hydroxide; and potassium hydroxide. Preferred bases are sodium hydroxide; potassium hydroxide; pyridine or triethylamine.

Suitable solvents for use in the third step of the above process include alcohols such as methanol, ethanol and isopropanol; water; tetrahydrofuran; dioxane; and acetonitrile. Preferred solvents are methanol or ethanol.

Examples of acids for use in the above process include concentrated hydrochloric acid or concentrated sulfuric acid.

The above process can be carried out at a temperature from about 0 °C to about 100 °C. Preferably, these reactions are carried out between about 0 °C and about 50 °C.

The following examples will further illustrate this invention, but are not intended to limit it in any way. In Table I, examples of the present invention are listed. Specific illustrative preparations of compounds of the invention are described following Table I.

## Table I

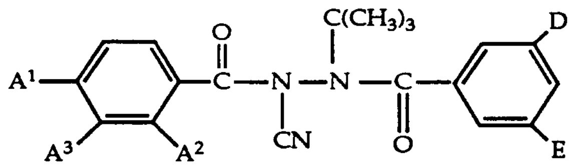

| Compound Number | $A^1$ | $A^2$ | $A^3$ | D | E |
|---|---|---|---|---|---|
| 1. | $CH_3$ | H | H | H | H |
| 2. | $CH_3$ | H | H | $CH_3$ | H |
| 3. | $CH_3$ | H | H | Cl | Cl |
| 4. | $CH_3$ | H | H | Cl | H |
| 5. | $CH_2CH_3$ | H | H | H | H |
| 6. | $CH_2CH_3$ | H | H | $CH_3$ | H |
| 7. | $CH_2CH_3$ | H | H | Br | H |
| 8. | $CH_2CH_2CH_3$ | H | H | H | H |
| 9. | $CH_2CH_3$ | H | H | $CH_2CH_3$ | H |
| 10. | $CH_3$ | H | H | Br | H |
| 11. | $CH_3$ | H | H | $CH_3$ | $CH_3$ |
| 12. | $CH_3$ | H | H | $CH_2CH_3$ | H |
| 13. | $CH_2CH_3$ | H | H | $CH_3$ | $CH_3$ |
| 14. | $CH(CH_3)_2$ | H | H | H | H |
| 15. | $CH(CH_3)_2$ | H | H | $CH_3$ | H |
| 16. | $CH_2CH_2CH_3$ | H | H | $CH_3$ | H |
| 17. | $CH_2CH_2CH_3$ | H | H | $CH_3$ | $CH_3$ |
| 18. | $CH(CH_3)_2$ | H | H | $CH_3$ | $CH_3$ |
| 19. | $CH_2CH_3$ | H | H | Cl | Cl |
| 20. | $CH_2CH_3$ | H | H | F | F |

| Compound Number | $A^1$ | $A^2$ | $A^3$ | D | E |
|---|---|---|---|---|---|
| 21. | $CH_2CH_3$ | H | H | Br | Br |
| 22. | $CH_2CH_3$ | H | H | Cl | H |
| 23. | H | $CH_3$ | $CH_3$ | $CH_3$ | H |
| 24. | H | $CH_3$ | Cl | $CH_3$ | H |
| 25. | H | $CH_3$ | Cl | H | H |
| 26. | H | Cl | Cl | H | H |
| 27. | H | Cl | Cl | $CH_3$ | H |
| 28. | H | $CH_3$ | $CH_3$ | $CH_3$ | $CH_3$ |
| 29. | H | $CH_3$ | $CH_3$ | Cl | H |
| 30. | H | $CH_3$ | Cl | $CH_3$ | $CH_3$ |
| 31. | H | $OCH_3$ | $OCH_3$ | H | H |
| 32. | H | $CH_3$ | Br | H | H |
| 33. | H | $CH_3$ | Br | $CH_3$ | H |
| 34. | H | $CH_3$ | F | H | H |
| 35. | H | $CH_3$ | F | $CH_3$ | H |
| 36. | H | Cl | Cl | $CH_3$ | $CH_3$ |
| 37. | H | Cl | Cl | Cl | Cl |
| 38. | H | Cl | Cl | Cl | H |
| 39. | H | F | F | $CH_3$ | H |
| 40. | H | F | F | $CH_3$ | $CH_3$ |
| 41. | H | $CH_3$ | $CH_3$ | H | H |
| 42. | H | Cl | $CH_3$ | H | H |
| 43. | H | Cl | $CH_3$ | $CH_3$ | H |
| 44. | H | Cl | $CH_3$ | $CH_3$ | $CH_3$ |
| 45. | H | Cl | $CH_3$ | Cl | Cl |
| 46. | H | Br | $CH_3$ | $CH_3$ | $CH_3$ |
| 47. | H | Br | $CH_3$ | $CH_3$ | H |
| 48. | H | Br | $CH_3$ | Cl | Cl |
| 49. | H | $CH_3$ | $CH_3$ | Cl | Cl |
| 50. | H | $CH_3$ | $CH_3$ | F | F |

| | | | | | |
|---|---|---|---|---|---|
| 51. | H | $CH_3$ | Cl | F | F |
| 52. | H | $CH_3$ | Cl | Cl | Cl |
| 53. | H | Br | $CH_3$ | Cl | H |
| 54. | H | Cl | $CH_3$ | Cl | H |
| 55. | H | Br | $CH_3$ | H | H |
| 56. | H | $CH_3$ | Cl | Cl | H |
| 57. | H | $CH_3$ | Cl | F | H |
| 58. | H | Cl | $OCH_3$ | $CH_3$ | $CH_3$ |
| 59. | H | $CH_3$ | Cl | $CH_3$ | $CH_3$ |
| 60. | H | F | F | H | H |
| 61. | H | Cl | $OCH_3$ | $CH_3$ | H |
| 62. | H | Cl | $OCH_3$ | H | H |
| 63. | H | $CH_2CH_3$ | Cl. | H | H |
| 64. | H | F | $CH_3$ | H | H |
| 65. | H | F | $CH_3$ | $CH_3$ | H |
| 66. | H | F | $CH_3$ | Cl | H |
| 67. | H | $CH_3$ | $CH_3$ | $CH_2CH_3$ | H |
| 68. | H | F | $CH_3$ | $CH_3$ | $CH_3$ |
| 69. | H | F | Cl | H | H |
| 70. | H | F | Cl | $CH_3$ | $CH_3$ |
| 71. | H | $CH_3$ | $CH_3$ | Br | Br |
| 72. | H | $CH_3$ | $CH_3$ | Br | H |
| 73. | H | $CH_3$ | $CH_3$ | $CH_3$ | Cl |
| 74. | H | $CH_3$ | $OCH_3$ | $CH_3$ | $CH_3$ |
| 75. | H | $CH_3$ | $OCH_3$ | H | $CH_3$ |

EXPERIMENTAL

**Example 13: N'-t-Butyl-N-cyano-N'-(3,5-dimethybenzoyl)-N-(4-ethylbenzoyl)-hydrazine**

*a. N'-t-Butyl-N'-(3,5-dimethylbenzoyl)-N-(4-ethylbenzoyl)-hydrazine*

To a suspension of 1.24 grams (g) of t-butylhydrazine hydrochloride (10 mmol) in toluene (30 ml) at room temperature was added dropwise a solution of 0.8 g of 50% aqueous sodium hydroxide (10 milliliters, ml) and the mixture was stirred for 15 minutes. The reaction mixture was then cooled to 5 °C and a solution of 4-ethylbenzoyl chloride (1.69 g, 10 mmol) in toluene (5 ml) and a solution of aqueous 50% sodium hydroxide (0.8 g, 10 mmol) were added dropwise simultaneously from separate addition funnels while maintaining the temperature at or below 10 °C. Following the addition, the reaction mixture was warmed to room temperature and stirred for 1 hour. The reaction mixture was diluted with toluene and washed with water. The organic layer was separated, dried over anhydrous magnesium sulfate, and the solvent was removed under vacuum to yield the monobenzoylated compound.

To a stirred solution of the monobenzoylated compound (2.19 g, 10 mmol) in toluene (30 ml) at 5 °C were added dropwise simultaneously from separate addition funnels, solutions of 3,5-dimethylbenzoyl chloride (1.69 g, 10 mmol) in toluene (5 ml) and aqueous 50% sodium hydroxide solution (0.8 g) while maintaining the temperature below 10 °C. Following the addition, the reaction mixture was warmed to room temperature and stirred for 1 hour. The mixture was then diluted with hexane and the precipitated product was isolated by filtration. The product was washed with water and hexane and dried. The crude product was recrystallized from ether/methanol to yield the product as a white powder, mp 181 °C.

*b. N'-t-Butyl-N-cyano-N'-(3,5-dimethybenzoyl)-N-(4-ethylbenzoyl)-hydrazine*

To a water bath cooled solution of N'-t-butyl-N'-(3,5-dimethylbenzoyl)-N-(4-ethylbenzoyl)hydrazine (3.52 grams (g), 10 mmol) in 150 milliliters (ml) of methylene chloride there was added tetrabutylammonium hydrogen sulfate (0.3 g, 0.88mmol) with stirring. Then 50% aqueous sodium hydroxide (5.2 ml) was added, followed by a dropwise addition of a solution of cyanogen bromide (1.6 g, 15 mmol) in methylene chloride (50 ml) at 20-25 °C. The resulting mixture was stirred for 1 hour. Then ice-cold distilled water (200 ml) was added and the organic layer was separated and washed with saturated aqueous sodium chloride and water. The organic layer was dried over anhydrous sodium carbonate, filtered and evaporated in vacuo to yield a yellow oil. Crystallization from hexane yielded 1.0 g of the desired product, mp 72-73 °C.

Following substantially the same procedure as described in Example 13a, the intermediates for Examples 1-7,9-20 and 26-75 described in Table 1 were also prepared, except the following caid chlorides were substituted for 4-ethylbenzoyl chloride: 4-methylbenzoyl chloride, 4-isopropylbenzoyl chloride, 4-n-propylbenzoyl chloride, 2,3-dichlorobenzoyl chloride, 2,3-dimethylbenzoyl chloride, 2-chloro-3-methylbenzoyl chloride, 2,3-dimethoxybenzoyl chloride, 3-bromo-2-methylbenzoyl chloride, 2-methyl-3-fluorobenzoyl chloride, 2,3-difluorobenzoyl chloride, 2-bromo-3-methylbenzoyl chloride, 2-methyl-3-chlorobenzoyl chloride, 2-chloro-3-methoxybenzoyl chloride, 2-ethyl-3-chlorobenzoyl chloride, 2-fluoro-3-methylbenzoylchloride, 2-methyl-3-methoxybenzoyl chloride or 2-fluoro-3-chlorobenzoyl chloride; and
the following acid chlorided were substituted for 2,3-dimethylbenzoyl chloride: benzoyl chloride, 3-methylbenzoyl chloride, 3,5-dichlorobenzoyl chloride, 3-chlorobenzoyl chloride, 3-bromobenzoyl chloride, 3-ethylbenzoyl chloride, 3,5-dimethylbenzoyl chloride, 3,5-difluorobenzoyl chloride, 3,5-dibromobenzoyl chloride, 3-chloro-5-methylbenzoyl chloride.

These intermediate compounds are reacted with cyanogen bromide following substantially the procedure described in Example 13b to obtain Examples 1-7,9-20 and 26-75.

**Example 23: N'-t-Butyl-N-cyano-N-(2,3-dimethybenzoyl)-N'-(3-methylbenzoyl)-hydrazine**

*a. N'-t-Butyl-N-(2,3-dimethylbenzoyl)-N'-(3-methylbenzoyl)-hydrazine*

Step 1

To a stirred suspension of t-butylhydrazine (51 g) in a mixture of dioxane and water (2:1) 150 ml) was added sodium hydroxide (32 g of a 50% aqueous solution). After 10 min., the solution was cooled to 50 °C and di-t-butyl-dicarbonate (42 g) was added dropwise so as to maintain the reaction temperature below 10 °C. The reaction mixture was warmed and stirred 2 hours at room temperature. The reaction was filtered, washed with water and dried to afford N-t-butyloxycarbonyl-N'-t-butylhydrazine (74 g) as a white crystalline solid. m.p. 69-71 °C.

Step 2

To a stirred solution of N-t-butyloxycarbonyl-N'-t-butylhydrazine (61 g) in toluene (120 ml) was added 3-methylbenzoyl chloride (45 g) and sodium hydroxide (31 g of a 50% aqueous sodium hydroxide solution) dropwise and simultaneously from a separate additionfunnel. After stirring for 1 hour at room temperature, the solid N-t-butyloxycarbonyl-N'-t-butyl-N'-(3-methylbenzoyl)hydrazine was filtered, washed with water, hexane and dried to afford the product.

Step 3

The N-t-butyloxycarbonyl-N'-t-butyl-N'-(3-methylbenzoyl)hydrazine ( 0.18 mol) was stirred at room temperature in a methanolic hydrochloric acid solution for 4 days. The reaction mixture was neutralized with

saturated aqueous sodium bicarbonate. The white precipitate was filtered, washed with water and dried in vacuo to give N'-t-butyl-N'-(3-methylbenzoyl)hydrazine.

Step 4

To a stirred mixture of N'-t-butyl-N'-(3-methylbenzoyl)hydrazine (1.0 g) in 15 ml toluene and aqueous sodium hydroxide (0.5 g of 50% NaOH) was added 2,3-dimethylbenzoyl chloride (2.0 g). After stirring for 2 hours, the product was isolated by filtration to give N'-t-butyl-N-(2,3-dimethybenzoyl)-N'-(3-methylbenzoyl)-hydrazine.

*b. N'-t-Butyl-N-cyano-N-(2,3-dimethybenzoyl)-N'-(3-methylbenzoyl)-hydrazine*

By following substantially the procedure of Example 13b, the desired product was prepared, mp 190-191.

By following substantially the procedure of Example 23a, the intermediates of Examples 8,24 and 25 were prepared except 4-n-propylbenzoyl chloride or 2-methyl-3-chlorobenzoyl chloride was used in place of 2,3-dimethyl benzoyl chloride; and N'-t-butyl-N'-benzoylhydrazine was used in place of N'-t-butyl-N'-3-methylbenzoylhydrazine.

By following substantially the procedure in Example 13b, Examples 8,24 and 25 are prepared.

The compounds of the invention are active by ingestion and contact action with the pests they control. In addition, they are unexpectedly active systemically within plants which support the pests to be controlled. Also, the compounds of the invention have an unexpectedly high solubility in organic solvents. These solubility characteristics enhance the ability to prepare liquid formulations which are expected to be more effective when applied to the target species.

On the basis of their strong initial pesticidal activity and excellent residual pesticidal activity, compounds according to the invention may be used in low dosages in controlling pests. The amount of dosage depends on a variety of factors, for example, the substance used, the kind of pest, the formulation used, the state of the crop infested with the pest and the prevailing weather conditions. In general, for the control of pests in agriculture and horticulture, a dosage corresponding to from about 0.01 grams to about 10 kilograms of the active substance per hectare may be used and from about 0.1 grams to about 200 grams per hectare of the active substance is preferred. The exact amount of dosage for a given situation can be routinely determined and depends on a variety of factors, for example, the substance used, the kind of pest, the formulation used, the state of the crop infested with the insect and the prevailing weather conditions.

The term "pesticidal" as employed in the specification and claims of this application is to be construed as any means which adversely affects the existence or growth of the target pest. Such means can compromise a complete killing action, eradication, arresting in growth, inhibition, reducing in number of any combination thereof. The term "control" as employed in the specification and claims of this application is to be construed as meaning "pesticidal" and protecting plants from pest damage. By "pesticidally effective amount" is meant that dosage of active substance sufficient to exert the desired pest "control".

The compounds of the present invention can be used in the form of compositions or formulations. Examples of the preparation of compositions and formulations can be found in the American Chemical Society publication "Pesticidal Formulation Research," (1969), Advances in Chemistry Series No. 86, written by Wade Van Valkenburg; and the Marcel Dekker, Inc. publication "Pesticide Formulations", (1973) edited by Wade Van Valkenburg. In these compositions and formulations, the active substance is mixed with conventional inert agronomically acceptable (i.e., plant compatible and/or pesticidally inert) pesticide diluents or extenders such as solid carrier material or liquid carrier material, of the type usable in conventional pesticide compositions or formulations. By "agronomically acceptable carrier" is meant any substance which can be used to dissolve, disperse or diffuse the active ingredient in the composition without impairing the active ingredient's effectiveness and which by itself has no significant detrimental effect on the soil, equipment, desirable plants, or agronomic environment. If desired, adjuvants such as surfactants, stabilizers, antifoam agents and antidrift agents may also be combined.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles. Wettable powders, pastes, flowables and emulsifiable concentrates are concentrated preparations which are diluted with water before or during use. Baits are preparations generally comprising a food or other substance attractive to insects, that includes at least one compound of the instant invention. The invert emulsions are mainly used for air application, where large areas are treated with a comparatively small

amount of preparation and may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional pesticide dispersible liquid diluent carriers and/or dispersible solid carriers optionally with the use of carrier vehicle assistants such as conventional pesticide surface-active agents, including emulsifying agents and/or dispersing agents, for example, when water is used as diluent, organic solvents may be added as auxiliary solvents.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists.

In the compositions of the invention, the active compound is present in an amount substantially between about 0.0001-99% by weight. For compositions suitable for storage or transportation, the amount of active ingredient is preferably between about 0.5-90% by weight, and more preferably between about 1-75% by weight of the mixture. Compositions suitable for direct application or field application generally contain the active compound in an amount substantially between about 0.0001-95%, preferably between about 0.0005-90% by weight, and more preferably between about 0.001-75% by weight of the mixture.

The active compounds can be applied as insecticide sprays by methods commonly employed, such as conventional high-gallonage hydraulic sprays, low gallonage sprays, ultra-low-volume sprays, airblast spray, aerial sprays, and dusts.

The present invention also contemplates methods of killing, combatting or controlling pests which compromises contacting pests with a combative or toxic amount (i.e. a pesticidally effective amount) of at least one active compound of the invention alone or together with a carrier vehicle (composition or formulation) as noted above. The term "contacting" as employed in the specification and claims means applying to at least one of (a) such pests and (b) the corresponding habit at thereof (i.e., the locus to be protected, for example, to a growing crop or to an area where a crop is to be grown) the active compound of this invention alone or as a constituent of a composition or formulation.

In addition to the aforementioned ingredients the preparations according to the invention may also contain other substances commonly used in preparations of this kind. For example, a lubricant, such as calcium stearate or magnesium stearate, may be added to a wettable powder or to a mixture to be granulated. Furthermore there may, for example, be added "adhesives" such as polyvinylalcohol-cellulose derivatives or other colloidal materials, such as casein, to improve the adherence of the pesticide to the surface to be protected.

Compositions and formulations according to the present invention may also include known pesticidal compounds. This expands the spectrum of activity of the preparation and may give rise to synergism.

The following known insecticidal, fungicidal and acaricidal compounds are suitable for use in such a combined preparation. Insecticides such as:

acephate, acethion, acetoxon, aldicarb, aldoxycarb, aldrin, allethrin, allyxycarb, alpha-cypermethrin, amidithion, amitraz, amlure, anethol, azethion, azinphos- ethyl, azinphos-methyl, azocyclotin, bacillus thuringiensis, BCPE, bendiocarb, bensultap, benzoximate, benzyl acetate, BHC, bifenthrin, binapacryl, bomyl, BPMC, bromophos, bromophos-ethyl, bromopropylate, bufencarb, buprofezin, butacarb, butocarboxim, butonate, butoxycarboxim, calcium arsenate, carbaryl, carbofuran, carbophenothion, carbosulfan, cartap, chlordane, chlordecone, chlordimeform, chlorfenethol, chlorfenson, chlorfensulphide, chlorfenvinphos, chlormephos, chlorobenzilate, chloropropylate, chlorphoxim, chlorpyrifos, chlorpyrifos methyl, chlorthiophos, clofentezine, CPCBS, CPMC, crotoxyphos, crufomate, cryolite, cufraneb, cyanofenphos, cyanophos, cyanthoate, cyfluthrin, cyhexatin, cypermethrin, cyphenothrin, cyromazine, DAEP, DDT, DDVP, deltamethrin, demeton, demeton-S-methyl, demeton-O-methyl, demeton-S, demeton-S-methyl sulfoxid, demephion-O, demephion-S, dialifor, diazinon, dicapthon, dicofol, dicrotophos, dieldrin, dienochlor, diflubenzuron, dihydrorotenone, dimefox, dimetan, dimethoate, dimethrin, dinex, dinitrophenol, dinobuton, dinocap, dioxabenzofos, dioxacarb, dioxathion, disparlure, disulfoton, DMCP, DNOC, d-trans allethrin, endosulfan, endothion, endrin, entice, EPBP, EPN, esfenvalerate, ethiofencarb, ethion, ethoate-methyl, ethoprop, etrimfos, fenamiphos, fenazaflor, fenbutatin-oxide, fenitrothion, fenoxycarb, fenpropathrin, fenson, fensulfothion, fenthion, fenvalerate, flubenzimine, flucythrinate, fluenethyl, flufenoxuron, fluvalinate, fonofos, formetanate hydrochloride, formothion, fosmethilan, fosthietan, furathiocarb, furethrin, grandlure, heptachlor, HETP, hexythiazox, hydramethylnon, hydroprene, IPSP, isazophos, isobenzan, isofenphos, isoprocarb, isoprothiolane, isothioate, isoxathion, jodfenphos, kinoprene, lead arsenate, leptophos, lethane, lindane, lythidathion, malathion, mazidox, mecarbam, mecarphon, menazon, mephosfolan, methamidophos,

methidathion, methiocarb, methomyl, methoprene, methoxychlor, methyl parathion, methyl phencapton, mevinphos, mexacarbate, MIPC, mirex, monocrotophos, MTMC, naled, nicotine, nonachlor, omethoate, ovex, oxamyl, oxydeprofs, oxydisulfoton, oxythioquinox, paraoxon, parathion, paris green, permethrin, perthane, phencapton, phenthoate, phorate, phosalone, phosfolan, phosmet, phosnichlor, phosphamidon, phoxim, pirimicarb, pirimiphos-ethyl, pirimiphos-methyl, plifenate, profenofos, promecarb, propargite, propetamphos, propoxur, prothidathion, prothiophos, prothoate, PTMD, pyridaben, pyridaphenthion, quinalphos, resmethrin, ronnell, rotenone, ryania, s-bioallethrin, salithion, schradan, sodium fluosilicate, sophamide, sulfotepp, sulprofos, tefluthrin, temephos, TEPP, terbufos, tetrachlorvinphos, tetradifon, tetramethrin, tetrasul, thallium sulfate, thiocarboxime, thiocyclam-hydrogenoxalate, thiometon, tolclofos-methyl, toxaphene, triazophos, trichlorfon, trichloronate, triflumuron, trimethacarb, vamidothion, and xylylcarb.

Fungicides which can be combined with the insecticides of this invention include:

(a) dithiocarbamate and derivatives such as ferbam, ziram, maneb, mancozeb,zineb, propineb, metham, thiram, the complex of zineb and polyethylene thiuram disulfide, dazomet, and mixtures of these with copper salts;

(b) nitrophenol derivatives such as dinocap, binapacryl, and 2-sec-butyl- 4,6-dinitrophenyl isopropyl carbonate;

(c) heterocyclic structures such as captan, folpet, glyodine, anilazine, ditalimfos, 4-butyl-1,2,4-triazole, 5-amino-1-[bis(dimethylamino) phosphinyl]-3-phenyl-1,2,4-triazole, etradiazole, dithianon, thioquinox, benomyl, thiabendazole, 4-(2-chlorophenylhydrazono)-3-methyl-5-isoxazolone, vinclozolin, iprodione, procymidone, triadimenol, triadimefon, bitertanol, prochloraz, fenasimol, bis-(p-chlorophenyl)-3-pyridinemethanol, bis-(p-chlorophenyl)- 5-pyrimidinemethanol, triarimol,flutriafol, flusilazole, propiconazole, ectaconazole, myclobutanil, alpha-[2-(4-chlorophenyl)ethyl]-alpha-phenyl-1H-1,2,4-triazole-1-propanenitrile, hexaconazole, cyproconazole, terbuconazole, diniconazole, fluoroimide, pyridine-2-thiol-1-oxide, 8-hydroxyquinoline sulfate and metal salts thereof, 2,3-dihydro-5-carboxanilido-6-methyl-1,4-oxathiin-4,4-dioxide, 2,3-dihydro-5-carboxanilido-6-methyl-1,4-oxathiin, cis-N-[(1,1,2,2-tetrachloroethyl)thiol]-4-cyclohexene-1,2-dicarboximide, cycloheximide, dehydroacetic acid, captafol, ethirimol, quinomethionate, D,L-methyl-N-(2,6-dimethylphenyl)- N-(2'-methoxyacetyl)alanine methyl ester, D,L-methyl-N-(2,6-dimethylphenyl)-N-chloroacetyl-D,L-2-aminobutyrolactone, D,L-N-(2,6-dimethylphenyl)-N-(phenylacetyl)alanine methyl ester, 5-methyl-5-vinyl-3-(3,5-dichlorophenyl)-2,4-dioxo-1,3-oxazolidine, 3-(3,5-dichlorophenyl) -5-methyl-5-(methoxymethyl)-1,3-oxazolidi-2,4-dione, 3-(3,5-dichlorophenyl)- 1-isopropylcarbamoylhydantoin, 2-cyano-[N-(ethylaminocarbonyl)-2- methoximino]acetamide, fenpropimorph, fenpropidine, 2,6-dimethyl-N- tridecylmorpholine, dodemorph, and triforine;

(d) miscellaneous halogenated fungicides such as chloranil, dichlone, chloroneb, tricamba, TCPN, dichloran, 2-chloro-1-nitropropane, polychloro-nitrobenzenes such as pentachloronitrobenzene (PCNB), and tetrafluorodi-chloroacetone;

(e) fungicidal antibiotics such as griseofulvin, kasugamycin, polyoxin, validamycin, and streptomycin;

(f) copper-based fungicides such as copper hydroxide, cuprous oxide, basic cupric chloride, basic copper carbonate, copper terephthalate, copper naphthenate and Bordeaux mixture; and

(g) miscellaneous fungicides such as dodine, phenylmercuric acetate, N-ethylmercuri-1,2,3,6-tetrahydro-3,6-endomethano-3,4,5,6,7,7-hexachloro- phthalimide, phenylmercuric monoethanol ammonium lactate, p-dimethyl-aminobenzene sodium sulfonate, methylisothiocyanate, 1-thiocyano-2,4-dinitrobenzene, 1-phenylthiosemicarbazide, nickel-containing compounds, calcium cyanamide, lime sulfur, thiophanate-methyl, flutolanil, edinophos, isoprothiolane, propenazole, and tricyclazole.

It has been found by biological evaluation that compounds according to the present invention have pesticidal activity and are capable of controlling larvae and adult forms of pests, especially insects from the orders Lepidoptera and Diptera, and most especially, insects from the order Lepidoptera. One skilled in the art will know how to determine the activity of a given compound against a given insect and the dosage required to obtain general or selective pesticidal effects. The compounds of the present invention affect the normal development of insects, particularly insects from the order Lepidoptera, by directly and/or indirectly influencing the moulting process.

As previously noted, the compounds of the present invention are particularly suitable for controlling plant destructive insects in crops of cultivated plants, such as, but not limited to, rice, cotton, vegetables, corn and other cereals and the like; forestry, such as, but not limited to, birch, spruce, pine, fir and the like; and ornamental plants, flowers and trees. Compounds of the present invention are also particularly suitable for controlling insects destructive to stored commodities such as seeds and the like; fruit crops, such as, but not limited to fruit and/or citrus trees, raspberry bushes and the like; and turf, such as, but not limited to, lawns, sod and the like. Additionally, the compounds of the invention do not appear to be toxic to beneficial insects such as honeybees and ladybugs.

In evaluating the pesticidal activity of the compounds of this invention, the following test procedures were employed.

A test solution containing 600 parts per million (ppm) was made by dissolving the test in a solvent (acetone:methanol, 1:1), adding water to give an acetone:methanol:water system of 5:5:90 and then a surfactant. A 1:1 mixture of an alkylarylpolyetheralcohol (sold under the trademark Triton® X-155) and a modified phthalic glycerol alkyl resin (sold under the trademark Triton® B-1956) was utilized at the equivalent of 1 ounce per gallon of test solution as a surfactant.

Initial evaluations were made on the following pest:

| Code Symbol | Common Name | Latin Name |
|---|---|---|
| SAW | Southern Armyworm | Spodoptera eridania |

For the foliar armyworm tests, individual bean (Phaseoulus limensis var. Woods' Prolific) leaves are placed on moistened pieces of filter paper in Petri dishes. The leaves are then sprayed with the test solution using a rotating turntable and allowed to dry. The dishes were infested with 10 third instar larvae of Southern armyworm. The dishes were then covered.

The percent mortality for the armyworm evaluations were determined 96 hours after treatment. Evaluations were based on a scale of 0-100 percent in which 0 equals no activity and 100 equals total kill.

The rotating turntable consisted of a fixed continuously operated spray nozzle under which targets were rotated at a fixed speed and distance. The distance from the nozzle was 15 inches. The nozzle was located 8 inches from the rotating shaft. The targets on individual platforms revolved around the shaft at 1 revolution per 20 seconds but only a brief portion of this time occured in the spray path. Targets passed only once under the nozzle and then were removed to drying hoods.

The nozzle used was a 1/4 JCO Spraying Systems (Wheaton, Illinois) air atomizing nozzle equipped with a No. 2850 fluid cap and No. 70 air cap. At the 10 psig air pressure used with liquid siphon feed 0.5 GPH (gallons per hour) were delivered in a round spray pattern with a 21° spray angle. Targets were misted with spray droplets to the point that the droplets coalesce to form a uniform thin film insufficient to drown test organisms.

All treatments were maintained at 75-80°F (24-27°C) under continuous fluorescent light in a well-ventilated room. The results of the initial pesticidal evaluations are given in Table III.

Table III

**Biological Evaluation**

Percent kill 600 ppm

Southern Armyworm

| Compound No. | Percent Kill |
|---|---|
| 13. | 100 |
| 23. | 100 |

## Claims

1. A compound of the formula

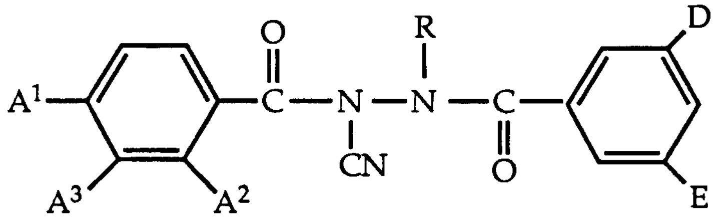

wherein R is t-butyl, methylneopentyl or neopentyl;

$A^1$ is hydrogen, methyl, ethyl, n-propyl or isopropyl;

when $A^1$ is hydrogen then $A^2$ and $A^3$ are each independently methyl, ethyl, methoxy, bromo, chloro or fluoro, and when $A^1$ is other than hydrogen, $A^2$ and $A^3$ are both hydrogen;

and D and E are each independently hydrogen, methyl, chloro, bromo or fluoro.

2. Compound according to claim 1 wherein $A^1$ is other than hydrogen.

3. Compound according to claim 2 wherein R is t-butyl.

4. Compound according to claim 2 or 3 wherein D is methyl and E is hydrogen or methyl.

5. Compound according to claim 4 which is N'-t-butyl-N-cyano-N'-(3,5-dimethylbenzoyl)-N-(4-ethylbenzoyl)hydrazine or N'-t-butyl-N-cyano-N'-(3,5-dimethylbenzoyl)-N-(4-methylbenzoyl)hydrazine.

6. Compound according to claim 3 wherein D and E are both hydrogen or both chloro.

7. Compound according to claim 6 which is N'-t-tbutyl-N-cyano-N'-(3,5-dichlorobenzoyl)-N-(4-methylbenzoyl)hydrazine or N'-t-butyl-N-cyano-N'-(3,5-dichlorobenzoyl)-N-(4-ethylbenzoyl)hydrazine.

8. Compound according to claim 3 which is N'-t-butyl-N-cyano-N'-benzoyl-N-(4-methylbenzoyl)hydrazine, N'-t-butyl-N-cyano-N-(4-methylbenzoyl)-N'-(3-methylbenzoyl)hydrazine, N'-t-butyl-N-cyano-N'-(3,5-dichlorobenzoyl)-N-(4-methylbenzoyl)hydrazine, N'-t-butyl-N-cyano-N'-(3-chlorobenzoyl)-N-(4-methylbenzoyl)hydrazine, N'-t-butyl-N-cyano-N'-benzoyl-N-(4-ethylbenzoyl)hydrazine, N'-t-butyl-N-cyano-N-(4-ethylbenzoyl)-N'-(3-methylbenzoyl)hydrazine N'-t-butyl-N-cyano-N-(3-bromobenzoyl)-N'-(4-ethylbenzoyl)hydrazine, N'-t-butyl-N-cyano-N'-benzoyl-N-(4-n-propylbenzoyl)hydrazine, N'-t-butyl-N-cyano-N-(4-ethylbenzoyl)-N'-(3-ethylbenzoyl)hydrazine, N'-t-butyl-N-cyano-N'-(3-bromobenzoyl)-N-(4-methylbenzoyl)hydrazine, N'-t-butyl-N-cyano-N'-(3,5-dimethylbenzoyl)-N-(4-methylbenzoy)hydrazine, N'-t-butyl-N-cyano-N'-(3-ethylbenzoyl)-N-(4-methylbenzoyl)hydrazine N'-t-butyl-N-cyano-N'-(3,5-dimethylbenzoyl)-N-(4-ethylbenzoyl)hydrazine, N'-t-butyl-N-cyano-N'-benzoyl-N-(4-isopropylbenzoyl)-hydrazine, N'-t-butyl-N-cyano-N'-(3-methylbenzoyl)-N-(4-n-propylbenzoyl)hydrazine, N'-t-butyl-N-cyano-N'-(3,5-dimethylbenzoyl)-N-(4-n-propylbenzoyl)hydrazine N'-t-butyl-N-cyano-N'-(3,5-dimethylbenzoyl)-N-(4-isopropylbenzoyl)hydrazine, or N'-t-butyl-N-cyano-N'-(3,5-difluorobenzoyl)-N-(4-ethylbenzoyl)-hydrazine.

9. Compound according to claim 1 wherein $A^1$ is hydrogen.

10. Compound according to claim 9 wherein R is t-butyl.

11. Compound according to claim 10 wherein $A^2$ and $A^3$ are each methyl, E is hydrogen or methyl, and D is hydrogen or methyl, preferably methyl.

12. Compound according to claim 9 which is N'-(t-butyl)-N-cyano-N-(2,3-dimethylbenzoyl)-N'-(3-methylbenzoyl)hydrazine, N'-(t-butyl)-N-cyano-N'-(3-methylbenzoyl)-N-(2-methyl-3-chlorobenzoyl)hydrazine, N'-(t-butyl)-N-cyano-N'-benzoyl-N-(2-methyl-3-chlorobenzoyl)hydrazine, N'-(t-butyl)-N-cyano-N'-benzoyl-N-(2,3-dichlorobenzoyl)hydrazine, N'-(t-butyl)-N-cyano-N-(2,3-dichlorobenzoyl)-N'-(3-methylbenzoyl)-hydrazine, N'-(t-butyl)-N-cyano-N-(2,3-dimethylbenzoyl)-N'-(3,5-dimethylbenzoyl)hydrazine, N'-(t-butyl)-N-cyano-N'-(3-chlorobenzoyl)-N-(2,3-dimethylbenzoyl)hydrazine, N'-(t-butyl)-N-cyano-N'-(3,5-dimethylbenzoyl)-N-(2-methyl-3-chlorobenzoyl)hydrazine, N'-(t-butyl)-N-cyano-N'-benzoyl-N-(2,3-dimethoxybenzoyl)hydrazine, N'-(t-butyl)-N-cyano-N'-benzoyl-N-(2-methyl-3-bromobenzoyl)hydrazine, N'-(t-butyl)-N-cyano-N'-(3-methylbenzoyl)-N-(2-methyl-3-bromobenzoyl)hydrazine, N'-(t-butyl)-N-cyano-N'-benzoyl-N-(2-methyl-3-fluorobenzoyl)hydrazine, N'-(t-butyl)-N-cyano-N'-(3,5-methylbenzoyl)-N-(2-methyl-3-fluorobenzoyl)hydrazine, N'-(t-butyl)-N-cyano-N-(2,3-dichlorobenzoyl)-N'-(3,5-dimethylbenzoyl)-hydrazine, N'-(t-butyl)-N-cyano-N-(2,3-dichlorobenzoyl)-N'-(3,5-dichlorobenzoyl)hydrazine, N'-(t-butyl)-N-cyano-N'-(3-chlorobenzoyl)-N-(2,3-dichlorobenzoyl)hydrazine, N'-(t-butyl)-N-cyano-N-(2,3-difluorobenzoyl)-N'-(3-methylbenzoyl)-hydrazine, N'-(t-butyl)-N-cyano-N-(2,3-difluorobenzoyl)-N'-(3,5-dimethylbenzoyl)hydrazine, N'-(t-butyl)-N-cyano-N'-benzoyl-N-(2,3-dimethylbenzoyl)hydrazine, N'-(t-butyl)-N-cyano-N'-benzoyl-N-(2-chloro-3-methylbenzoyl)hydrazine, N'-(t-butyl)-N-cyano-N-(2-chloro-3-methylbenzoyl)-N'-(3-methylbenzoyl)hydrazine, N'-(t-butyl)-N-cyano-N-(2-chloro-3-methylbenzoyl)-N'-(3,5-dimethylbenzoyl)hydrazine, N'-(t-butyl)-N-cyano-N-(2-chloro-3-methylbenzoyl)-N'-(3,5-dichlorobenzoyl)hydrazine, N'-(t-butyl)-N-cyano-N-(2-bromo-3-methylbenzoyl)-N'-(3,5-dimethylbenzoyl)hydrazine, N'-(t-butyl)-N-cyano-N-(2-bromo-3-methylbenzoyl)-N'-(3-methylbenzoyl)hydrazone, N'-(t-butyl)-N-cyano-N-(2-bromo-3-methylbenzoyl)-N'-(3,5-dichlorobenzoyl)hydrazine, N'-(t-butyl)-N-cyano-N'-(3,5-dichlorobenzoyl)-N-(2,3-dimethylbenzoyl)-hydrazine, N'-(t-butyl)-N-cyano-N'-(3,5-difluorobenzoyl)-N-(2,3-dimethylbenzoyl)-hydrazine, N'-(t-butyl)-N-cyano-N'-(3,5-difluorobenzoyl)-N-(2,-methyl-3-chlorobenzoyl)hydrazine, N'-(t-butyl)-N-cyano-N'-(3,5-dichlorobenzoyl)-N-(2-methyl-3-chlorobenzoyl)hydrazine, N'-(t-butyl)-N-cyano-N-(2-bromo-3-methylbenzoyl)-N'-(3-chlorobenzoyl)hydrazine, N'-(t-butyl)-N-cyano-N-(3-chlorobenzoyl)-N-(2-chloro-3-methylbenzoyl)hydrazine, N'-(t-butyl)-N-cyano-N'-benzoyl-N-(2-bromo-3-methylbenzoyl)-hydrazine, N'-(t-butyl)-N-cyano-N'-(3-chlorobenzoyl)-N-(2-methyl-3-chlorobenzoyl)hydrazine, N'-(t-butyl)-N-cyano-N-(3-fluorobenzoyl)-N-(2-methyl-3-chlorobenzoyl)hydrazine, N'-t-butyl-N-cyano-N'-(3,5-dimethylbenzoyl)-N-(3-methoxy-2-methylbenzoyl)hydrazine or N'-t-butyl-N-cyano-N-(3-methoxy-2-methylbenzoyl)-N'-(3-methylbenzoyl)hydrazine.

**13.** An insecticidal composition comprising a compound according to any preceding claim in an insecticidally active amount, preferably 0.0001-95% by weight, more preferably 0.0005-90% by weight, and most preferably 0.001-75% by weight of the composition, and an agronomically acceptable carrier.

**14.** A method for controlling insects comprising contacting the insects or the habitation thereof with an insecticidally-effective amount of a compound or composition according to any preceding claim.

**15.** Method according to claim 14 wherein the insects are the order of Lepidoptera or Diptera, preferably Lepidoptera.

**16.** Method according to claim 14 or 15 wherein the compound or composition is applied in an amount of from 0.01 to about 10 kilograms, preferably from 0.1 to 200g, per hectare.

**17.** Process for producing a compound as defined in any of claims 1 to 12 comprising the steps of:

(i) either a) reacting a substituted or unsubstituted benzoyl halide with an alkyl hydrazine or acid salt thereof in the presence of base, and then reacting the resultant monobenzoxylated alkylhydrazine with a further substituted or unsubstituted benzoyl halide in the presence of base to yield an N, N'-dibenzoyl-N'-alkylhydrazine,

or b) reacting an alkylhydrazine or acid salt thereof with a dicarbonate in the presence of base, reacting the resultant alkoxycarbonyl-N'-alkylhydrazine with a substituted or unsubstituted benzoyl halide in the presence of base, reacting the then resultant alkoxycarbonyl-N'-alkyl-N'-benzoylhydrazine with an acid, and then reacting the resultant N'-alkyl-N'-benzoylhydrazine with a further subsituted or unsubsituted benzoy halide in the presence of base to yield an N,N'-dibenzoyl-N'-alkylhydrazine,

all the above reactions being carried out in a substantially inert solvent;

(ii) reacting said N,N'-dibenzoyl-N'-alkylhydrazine with an alkali metal hydride and cyanogen halide, to yield said compound.

## Patentansprüche

1. Verbindung der Formel

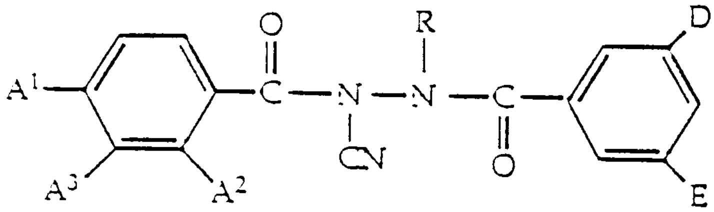

in der R t-Butyl, Methylneopentyl oder Neopentyl ist, $A^1$ ist Wasserstoff, Methyl, Ethyl, n-Propyl oder Isopropyl, wobei dann, wenn $A^1$ Wasserstoff ist, $A^2$ und $A^3$ jeweils unabhängig Methyl, Ethyl, Methoxy, Brom, Chlor oder Fluor sind, und wenn $A^1$ nicht Wasserstoff ist, sind $A^2$ und $A^3$ beide Wasserstoff, und D und E sind jeweils unabhängig Wasserstoff, Methyl, Chlor, Brom oder Fluor.

2. Verbindung nach Anspruch 1,
**dadurch gekennzeichnet**,
daß $A^1$ kein Wasserstoff ist.

3. Verbindung nach Anspruch 2,
**dadurch gekennzeichnet**,
daß R t-Butyl ist.

4. Verbindung nach Anspruch 2 oder 3,
**dadurch gekennzeichnet**,
daß D Methyl und E Wasserstoff oder Methyl sind.

5. Verbindung nach Anspruch 4,
**dadurch gekennzeichnet**,
daß sie N'-t-Butyl-N-cyano-N'-(3,5-dimethylbenzoyl)-N-(4-ethylbenzoyl)hydrazin oder N'-t-Butyl-N-cyano-N'-(3,5-dimethylbenzoyl)-N-(4-Methylbenzoyl)hydrazin ist.

6. Verbindung nach Anspruch 3,
**dadurch gekennzeichnet**,
daß D und E beide Wasserstoff oder beide Chlor sind.

7. Verbindung nach Anspruch 6,
**dadurch gekennzeichnet**,
daß sie N'-t-Butyl-N-cyano-N'-(3,5-dichlorbenzoyl)-N-(4-methylbenzoyl)hydrazin oder N'-t-Butyl-N-cyano-N'-(3,5-dichlorbenzoyl)-N-(4-ethylbenzoyl)hydrazin ist.

8. Verbindung nach Anspruch 3,
**dadurch gekennzeichnet**,
daß Sie N'-t-Butyl-N-cyano-N'-benzoyl-N-(4-methylbenzoyl)hydrazin, N'-t-Butyl-N-cyano-N-(4-methylbenzoyl)-N'-(3-methylbenzoyl)hydrazin, N'-t-Butyl-N-cyano-N'-(3,5-dichlorbenzoyl)-N-(4-methylbenzoyl)-hydrazin, N'-t-Butyl-N-cyano-N'-(3-chlorbenzoyl)-N-(4-methylbenzoyl)hydrazin, N'-t-Butyl-N-cyano-N'-benzoyl-N-(4-ethylbenzoyl)hydrazin, N'-t-Butyl-N-cyano-N-(4-ethylbenzoyl)-N'-(3-methylbenzoyl)-hydrazin, N'-t-Butyl-N-cyano-N-(3-brombenzoyl)-N'-(4-ethylbenzoyl)hydrazin, N'-t-Butyl-N-cyano-N'-benzoyl-N-(4-n-propylbenzoyl)hydrazin, N'-t-Butyl-N-cyano-N-(4-ethylbenzoyl)-N'-(3-ethylbenzoyl)-hydrazin, N'-t-Butyl-N-cyano-N'-(3-brombenzoyl)-N-(4-methylbenzoyl)hydrazin, N'-t-Butyl-N-cyano-N'-(3,5-dimethylbenzoyl)-N-(4-methylbenzoyl)hydrazin, N'-t-Butyl-N-cyano-N'-(3-ethylbenzoyl)-N-(4-methylbenzoyl)hydrazin, N'-t-Butyl-N-cyano-N'-(3,5-dimethylbenzoyl)-N-(4-ethylbenzoyl)hydrazin, N'-t-Butyl-N-cyano-N'-benzoyl-N-(4-isopropylbenzoyl)hydrazin, N'-t-Butyl-N-cyano-N'-(3-methylbenzoyl)-N-(4-n-propylbenzoyl)hydrazin, N'-t-Butyl-N-cyano-N'-(3,5-dimethylbenzoyl)-N-(4-n-propylbenzoyl)hydrazin, N'-t-Butyl-N-cyano-N'-(3,5-dimethylbenzoyl)-N-(4-isopropylbenzoyl)hydrazin, oder N'-t-Butyl-N-cyano-N'-(3,5-difluorbenzoyl)-N-(4-ethylbenzoyl)hydrazin ist.

**9.** Verbindung nach Anspruch 1,
**dadurch gekennzeichnet**,
daß $A^1$ Wasserstoff ist.

**10.** Verbindung nach Anspruch 9,
**dadurch gekennzeichnet**,
daß R t-Butyl ist.

**11.** Verbindung nach Anspruch 10,
**dadurch gekennzeichnet**,
daß $A^2$ und $A^3$ jeweils Methyl sind, E Wasserstoff oder Methyl ist und D Wasserstoff oder Methyl, vorzugsweise Methyl ist.

**12.** Verbindung nach Anspruch 9,
**dadurch gekennzeichnet**,
daß sie N'-(t-Butyl)-N-cyano-N-(2,3-dimethylbenzoyl)-N'-(3-methylbenzoyl)hydrazin, N'-(t-Butyl)-N-cyano-N'-(3-methylbenzoyl)-N-(2-methyl-3-chlorbenzoyl)hydrazin, N'-(t-Butyl)-N-cyano-N'-benzoyl-N-(2-methyl-3-chlorbenzoyi)hydrazin, N'-(t-Butyl)-N-cyano-N'-benzoyl-N-(2,3-dichlorbenzoyl)hydrazin, N'-(t-Butyl)-N-cyano-N-(2,3-dichlorbenzoyl)-N'-(3-methlybenzoyl)hydrazin, N'-(t-Butyl)-N-cyano-N-(2,3-dimethylbenzoyl)-N'-(3,5-dimethylbenzoyl)hydrazin, N'-(t-Butyl)-N-cyano-N'-(3-chlorbenzoyl)-N-(2,3-dimethylbenzoyl)hydrazin, N'-(t-Butyl)-N-cyano-N'-(3,5-dimethylbenzoyl)-N-(2-methyl-3-chlorbenzoyl)-hydrazin, N'-(t-Butyl)-N-cyano-N'-benzoyl-N-(2,3-dimethoxybenzoyl)hydrazin, N'-(t-Butyl)-N-cyano-N'-benzoyl-N-(2-methyl-3-brombenzoyl)hydrazin, N'-(t-Butyl)-N-cyano-N'-(3-methylbenzoyl)-N-(2-methyl-3-brombenzoyl)hydrazin, N'-(t-Butyl)-N-cyano-N'-benzoyl-N-(2-methyl-3-fluorbenzoyl)hydrazin, N'-(t-Butyl)-N-cyano-N'-(3,5-methylbenzoyl)-N-(2-methyl3-fluorbenzoyl)hydrazin, N'-(t-Butyl)-N-cyano-N-(2,3-dichlorbenzoyl)-N'-(3,5-dimethylbenzoyl)hydrazin, N'-(t-Butyl)-N-cyano-N-(2,3-dichlorbenzoyl)-N'-(3,5-dichlorbenzoyl)hydrazin, N'-(t-Butyl)-N-cyano-N'-(3-chlorbenzoyl)-N-(2,3-dichlorbenzoyl)hydrazin, N'-(t-Butyl)-N-cyano-N-(2,3-difluorbenzoyl)-N'-(3-methylbenzoyl)hydrazin, N'-(t-Butyl)-N-cyano-N-(2,3-difluorbenzoyl)-N'-(3,5-dimethylbenzoyl)hydrazin, N'-(t-Butyl)-N-cyano-N'-benzoyl-N-(2,3-dimethylbenzoyl)-hydrazin, N'-(t-Butyl)-N-cyano-N'-benzoyl-N-(2-chlor-3-methylbenzoyl)hydrazin, N'-(t-Butyl)-N-cyano-N-(2-chlor-3-methylbenzoyl)-N'-(3-methylbenzoyl)hydrazin, N'-(t-Butyl)-N-cyano-N-(2-chlor-3-methylbenzoyl)-N'-(3,5-dimethylbenzoyl)hydrazin, N'-(t-Butyl)-N-cyano-N-(2-chlor-3-methylbenzoyl)-N'-(3,5-dichlorbenzoyl)hydrazin, N'-(t-Butyl)-N-cyano-N-(2-brom-3-methylbenzoyl)-N'-(3,5-dimethylbenzoyl)hydrazin, N'-(t-Butyl)-N-cyano-N-(2-brom-3-methylbenzoyl)-N'-(3-methylbenzoyl)hydrazin, N'-(t-Butyl)-N-cyano-N-(2-brom-3-methylbenzoyl)-N'-(3,5-dichlorbenzoyl)hydrazin, N'-(t-Butyl)-N-cyano-N'-(3,5-dichlorbenzoyl)-N-(2,3-dimethylbenzoyl)hydrazin, N'-(t-Butyl)-N-cyano-N'-(3,5-difluorbenzoyl)-N-(2,3-dimethylbenzoyl)-hydrazin, N'-(t-Butyl)-N-cyano-N'-(3,5-difluorbenzoyl)-N-(2-methyl-3-chlorbenzoyl)hydrazin, N'-(t-Butyl)-N-cyano-N'-(3,5-dichlorbenzoyl)-N-(2-methyl-3-chlorbenzoyl)hydrazin, N'-(t-Butyl)-N-cyano-N-(2-brom-3-methylbenzcyl)-N'-(3-chlorbenzoyl)hydrazin,N'-(t-Butyl)-N-cyano-N-(3-chlorbenzoyl)-N-(2-chlor-3-methylbenzoyl)hydrazin, N'-(t-Butyl)-N-cyano-N'-benzoyl-N-(2-brom-3-methylbenzoyl)hydrazin, N'-(t-Butyl)-N-cyano-N'-(3-chlorbenzoyl)-N-(2-methyl-3-chlorbenzoyl)hydrazin, N'-(t-Butyl)-N-cyano-N-(3-fluorbenzoyl)-N-(2-methyl-3-chlorbenzoyl)hydrazin, N'-t-Butyl-N-cyano-N'-(3,5-dimethylbenzoyl)-N-(3-methoxy-2-methylbenzoyl)hydrazin oder N'-t-Butyl-N-cyano-N-(3-methoxy-2-methylbenzoyl)-N'-(3-methylbenzoyl)-hydrazin ist.

**13.** Insektizide Zusammensetzung, enthaltend eine Verbindung nach einem der vorstehenden Ansprüche in einer insektizid wirksamen Menge, vorzugsweise 0,0001-95 Gew.-%, bevorzugter 0,0005-90 Gew-%, am meisten bevorzugt 0,001-75 Gew.-% der Zusammensetzung, und einen landwirtschaftlich verträglichen Träger.

**14.** Verfahren zum Steuern von Insekten durch Inberührungbringen der Insekten oder ihrer Lebensumgebung mit einer insektizid wirksamen Menge einer Verbindung oder Zusammensetzung nach einem der vorstehenden Ansprüche.

**15.** Verfahren nach Anspruch 14,
**dadurch gekennzeichnet**,

daß die Insekten der Gruppe Lepidoptera oder Diptera, vorzugsweise Lepidoptera, angehören.

**16.** Verfahren nach Anspruch 14 oder 15,
**dadurch gekennzeichnet**,
daß die Verbindung oder Zusammensetzung in einer Menge von 0,01 bis etwa 10 kg, vorzugsweise von 0,1 bis 200 g, pro Hektar aufgebracht wird.

**17.** Verfahren zum Herstellen einer Verbindung nach einem der Ansprüche 1-12 durch die Schritte:
( i) entweder a) Umsetzen eines substituierten oder nicht substituierten Benzoylhalids mit einem Alkylhydrazin oder Säuresalz desselben in Gegenwart einer Base und dann Umsetzen des erhaltenen monobenzoxylierten Alkylhydrazins mit einem weiteren substituierten oder nicht substituierten Benzoylhalids in Gegenwart einer Base, um ein N,N'-Dibenzoyl-N'-alkylhydrazin auszubilden,
oder b) Umsetzen eines Alkylhydrazins oder Säuresalzes desselben mit einem Dicarbonat in Gegenwart einer Base, Umsetzen des erhaltenen Alkoxycarbonyl-N'-alkylhydrazins mit einem substituierten oder nicht substituierten Benzoylhalids in Gegenwart einer Base, anschließendes Umsetzen des erhaltenen Alkoxycarbonyl-N'-alkyl-N'-benzoylhydrazins mit einer Säure und dann Umsetzen des erhaltenen N'-Alkyl-N'-benzoylhydrazins mit einem weiteren substituierten oder nicht substituierten Benzoylhalid in Gegenwart einer Base, um ein N,N'-Dibenzoyl-N'-alkylhydrazin auszubilden, wobei alle der vorstehenden Reaktionen in einem im wesentlichen inerten Lösemittel ausgeführt werden,
(ii) Umsetzen des N,N'-Dibenzoyl-N'-alkylhydrazins mit einem Alkalihydrid und Cyanhalid, um die Verbindung auszubilden.

## Revendications

**1.** Composé de formule

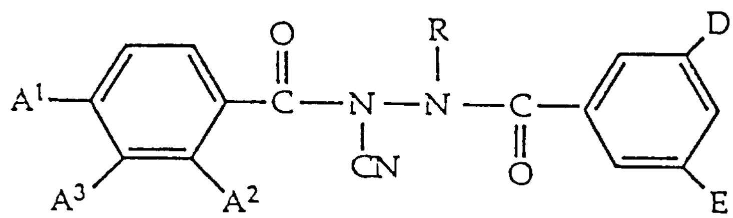

dans laquelle R est un radical tert-butyle, méthylnéopentyle ou néopentyle ;
$A^1$ est un atome d'hydrogène ou un radical méthyle, éthyle, n-propyle ou isopropyle ;
quand $A^1$ est un atome d'hydrogène, $A^2$ et $A^3$ sont chacun, indépendamment l'un de l'autre, un radical méthyle, éthyle, méthoxy, bromo, chloro ou fluoro ; et quand $A^1$ est différent d'un atome d'hydrogène, $A^2$ et $A^3$ sont chacun un atome d'hydrogène ;
et D et E, chacun indépendamment l'un de l'autre, sont un atome d'hydrogène ou un radical méthyle, chloro, bromo ou fluoro.

**2.** Composé selon la revendication 1, dans lequel $A^1$ n'est pas un atome d'hydrogène.

**3.** Composé selon la revendication 2, dans lequel R est un radical tert-butyle.

**4.** Composé selon la revendication 2 ou 3, dans lequel D est un radical méthyle et E est un atome d'hydrogène ou un radical méthyle.

**5.** Composé selon la revendication 4, qui est la N'-tert-butyl-N-cyano-N'-(3,5-diméthylbenzoyl)-N-(4-éthylbenzoyl)hydrazine ou la N'-tert-butyl-N-cyano-N'-(3,5-diméthylbenzoyl)-N-(4-méthylbenzoyl)-hydrazine.

**6.** Composé selon la revendication 3, dans lequel D et E sont chacun un atome d'hydrogène ou sont chacun un radical chloro.

7. Composé selon la revendication 6, qui est la N'-tert-butyl-N-cyano-N'-(3,5-dichlorobenzoyl)-N-(4-méthylbenzoyl)hydrazine ou la N'-tert-butyl-N-cyano-N'-(3,5-dichlorobenzoyl)-N-(4-éthylbenzoyl)hydrazine.

8. Composé selon la revendication 3, qui est la N'-tert-butyl-N-cyano-N'-benzoyl-N-(4-méthylbenzoyl)-hydrazine, la N'-tert-butyl-N-cyano-N-(4-méthylbenzoyl)-N'-(3-méthylbenzoyl)hydrazine, la N'-tert-butyl-N-cyano-N'-(3,5-dichlorobenzoyl)-N-(4-méthylbenzoyl)hydrazine, la N'-tert-butyl-N-cyano-N'-(3-chlorobenzoyl-N-(4-méthylbenzoyl)hydrazine, la N'-tert-butyl-N-cyano-N'-benzoyl-N-(4-éthylbenzoyl)-hydrazine,la N'-tert-butyl-N-cyano-N-(4-éthylbenzoyl)-N'-(3-méthylbenzoyl)hydrazine, la N'-tert-butyl-N-cyano-N-(3-bromobenzoyl)-N'-(4-éthylbenzoyl)hydrazine, la N'-tert-butyl-N-cyano-N'-benzoyl-N-(4-n-propylbenzoyl)hydrazine, la N'-tert-butyl-N-cyano-N-(4-éthylbenzoyl)-N'-(3-éthylbenzoyl)hydrazine, la N'-tert-butyl-N-cyano-N'-(3-bromobenzoyl)-N-(4-méthylbenzoyl)hydrazine, la N'-tert-butyl-N-cyano-N'-(3,5-diméthylbenzoyl)-N-(4-méthylbenzoyl)hydrazine, la N'-tert-butyl-N-cyano-N'-(3-éthylbenzoyl)-N-(4-méthylbenzoyl)hydrazine, la N'-tert-butyl-N-cyano-N'-(3,5-diméthylbenzoyl)-N-(4-éthylbenzoyl)hydrazine, la N'-tert-butyl-N-cyano-N'-benzoyl-N-(4-isopropylbenzoyl)hydrazine, la N'-tert-butyl-N-cyano-N'-(3-méthylbenzoyl)-N-(4-n-propylbenzoyl)hydrazine, la N'-tert-butyl-N-cyano-N'-(3,5-diméthylbenzoyl)-N-(4-n-propylbenzoyl)-hydrazine, la N'-tert-butyl-N-cyano-N'-(3,5-diméthylbenzoyl)-N-(4-isopropylbenzoyl)-hydrazine ou la N'-tert-butyl-N-cyano-N'-(3,5-difluorobenzoyl)-N-(4-éthylbenzoyl)hydrazine.

9. Composé selon la revendication 1, dans lequel $A^1$ est un atome d'hydrogène.

10. Composé selon la revendication 9, dans lequel R est un radical tert-butyle.

11. Composé selon la revendication 10, dans lequel $A^2$ et $A^3$ sont chacun un radical méthyle, E est un atome d'hydrogène ou un radical méthyle, et D est un atome d'hydrogène ou le radical méthyle, de préférence méthyle.

12. Composé selon la revendication 9, qui est la N'-(tert-butyl)-N-cyano-N-(2,3-diméthylbenzoyl)-N'-(3-méthylbenzoyl)hydrazine, la N'-(tert-butyl)-N-cyano-N'-(3-méthylbenzoyl)-N-(2-méthyl-3-chlorobenzoyl)-hydrazine, la N'-(tert-butyl)-N-cyano-N'-benzoyl-N-(2-méthyl-3-chlorobenzoyl)hydrazine, la N'-(tert-butyl)-N-cyano-N'-benzoyl-N-(2,3-dichlorobenzoyl)hydrazine, la N'-(tert-butyl)-N-cyano-N-(2,3-dichlorobenzoyl)-N'-(3-méthylbenzoyl)hydrazine, la N'-(tert-butyl)-N-cyano-N-(2,3-diméthylbenzoyl)-N'-(3,5-diméthylbenzoyl)hydrazine, la N'-(tert-butyl)-N-cyano-N'-(3-chlorobenzoyl)-N-(2,3-diméthylbenzoyl)hydrazine, la N'-(tert-butyl)-N-cyano-N'-(3,5-diméthylbenzoyl)-N-(2-méthyl-3-chlorobenzoyl)hydrazine, la N'-(tert-butyl)-N-cyano-N'-benzoyl-N-(2,3-diméthoxybenzoyl)hydrazine, la N'-(tert-butyl)-N-cyano-N'-benzoyl-N-(2-méthyl-3-bromobenzoyl)hydrazine, la N'-(tert-butyl)-N-cyano-N'-(3-méthylbenzoyl)-N-(2-méthyl-3-bromobenzoyl)hydrazine, la N'-(tert-butyl)-N-cyano-N'-benzoyl-N-(2-méthyl-3-fluorobenzoyl)hydrazine, la N'-(tert-butyl)-N-cyano-N'-(3,5-méthylbenzoyl)-N-(2-méthyl-3-fluorobenzoyl)hydrazine, la N'-(tert-butyl)-N-cyano-N-(2,3-dichlorobenzoyl)-N'-(3,5-diméthylbenzoyl)hydrazine, la N'-(tert-butyl)-N-cyano-N-(2,3-dichlorohenzoyl)-N'-(3,5-dichlorobenzoyl)hydrazine, la N'-(tert-butyl)-N-cyano-N'-(3-chlorobenzoyl)-N-(2,3-dichlorobenzoyl)hydra-zine, la N'-(tert-butyl)-N-cyano-N-(2,3-difluorobenzoyl)-N'-(3-méthylhenzoyl)-hydrazine, la N'-(tert-butyl)-N-cyano-N-(2,3-difluorobenzoyl)-N'-(3,5-diméthylbenzoyl)-hydrazine, la N'-(tert-butyl)-N-cyano-N'-benzoyl-N-(2,3-diméthylbenzoyl)hydrazine, la N'-(tert-butyl)-N-cyano-N'-benzoyl-N-(2-chloro-3-méthylbenzoyl)hydrazine, la N'-(tert-butyl)-N-cyano-N-(2-chloro-3-méthylbenzoyl)-N'-(3-méthylbenzoyl)hydrazine, la N'-(tert-butyl)-N-cyano-N-(2-chloro-3-méthylbenzoyl)-N'-(3,5-diméthylbenzoyl)hydrazine, la N'-(tert-butyl)-N-cyano-N-(2-chloro-3-méthylbenzoyl)-N'-(3,5-dichlorobenzoyl)-hydrazine, la N'-(tert-butyl)-N-cyano-N-(2-bromo-3-méthylbenzoyl)-N'-(3,5-diméthylbenzoyl)hydrazine, la N'-(tert-butyl)-N-cyano-N-(2-bromo-3-méthylbenzoyl)-N'-(3-méthylbenzoyl)hydrazine, la N'-(tert-butyl)-N-cyano-N-(2-bromo-3-méthylbenzoyl)-N'-(3,5-dichlorobenzoyl)hydrazine, la N'-(tert-butyl)-N-cyano-N'-(3,5-dichlorobenzoyl)-N-(2,3-diméthylbenzoyl)hydrazine, la N'-(tert-butyl)-N-cyano-N'-(3,5-difluorobenzoyl)-N-(2,3-diméthylbenzoyl)hydrazine, la N'-(tert-butyl)-N-cyano-N'-(3,5-difluoro-benzoyl)-N-(2-méthyl-3-chlorobenzoyl)hydrazine, la N'-(tert-butyl)-N-cyano-N'-(3,5-dichlorobenzoyl)-N-(2-méthyl-3-chlorobenzoyl)hydrazine, la N'-(tert-butyl)-N-cyano-N-(2-bromo-3-méthylbenzoyl)-N'-(3-chlorobenzoyl)hydrazine, la N'-(tert-butyl)-N-cyano-N-(3-chlorobenzoyl)-N-(2-chloro-3-méthylbenzoyl)hydrazine, la N'-(tert-butyl)-N-cyano-N'-benzoyl-N-(2-bromo-3-méthylbenzoyl)hydrazine, la N'-(tert-butyl)-N-cyano-N'-(3-chlorobenzoyl)-N-(2-méthyl-3-chlorobenzoyl)hydrazine, la N'-(tert-butyl)-N-cyano-N-(3-fluorobenzoyl)-N-(2-méthyl-3-chlorobenzoyl)hydrazine, la N'-(tert-butyl)-N-cyano-N'-(3,5-diméthylbenzoyl)-N-(3-méthoxy-2-méthylbenzoyl)hydrazine ou la N'-(tert-butyl)-N-cyano-N-(3-méthoxy-2-méthylbenzoyl)-N'-(3-méthylbenzoyl)-hydrazine.

**13.** Composition insecticide comprenant un composé selon l'une quelconque des revendications précédentes en une quantité à activité insecticide, de préférence de 0,0001 à 95 % en poids, de préférence de 0,0005 à 90 % en poids et tout spécialement de 0,001 à 75 % en poids par rapport à la composition, et un support acceptable d'un point de vue agronomique.

**14.** Procédé pour maîtriser des insectes, qui consiste à mettre en contact les insectes ou leur habitation avec une quantité à effet insecticide d'un composé ou d'une composition selon l'une quelconque des revendications précédentes.

**15.** Procédé selon la revendication 14, dans lequel les insectes appartiennent à l'ordre des Lépidoptères ou des Diptères, de préférence des Lépidoptères.

**16.** Procédé selon la revendication 14 ou 15, dans lequel le composé ou la composition est appliqué selon une quantité de 0,01 à environ 10 kg, de préférence de 0,1 à 200 g par hectare.

**17.** Procédé pour préparer un composé tel que défini selon l'une quelconque des revendications 1 à 12, qui comprend les étapes consistant :

(i) a) à faire réagir un halogénure de benzoyle substitué ou non substitué avec une alkylhydrazine ou l'un de ses sels acides en présence d'une base, puis à faire réagir l'alkylhydrazine monobenzoxylée obtenue avec un halogénure de benzoyle supplémentaire substitué, ou non substitué, en présence d'une base, pour donner une N,N'-dibenzoyl-N'-alkylhydrazine, ou bien

b) à faire réagir une alkylhydrazine ou l'un de ses sels acides avec un dicarbonate en présence d'une base, à faire réagir l'alcoxycarbonyl-N'-alkylhydrazine obtenue avec un halogénure de benzoyle substitué ou non substitué en présence d'une base, à faire réagir avec un acide l'alcorycarbonyle-N'-alkyl-N'-benzoylhydrazine ainsi obtenue, puis à faire réagir la N'-alkyl-N'-benzoylhydrazine obtenue avec un halogénure de benzoyle supplémentaire substitué ou non substitué, en présence d'une base, pour donner une N,N'-dibenzoyl-N'-alkylhydrazine,

toutes les réactions ci-dessus étant mises en oeuvre dans un solvant essentiellement inerte ;

(ii) à faire réagir ladite N,N'-dibenzoyl-N'-alkylhydrazine avec un hydrure d'un métal alcalin et un halogénure de cyanogène, pour donner le composé recherché.